(19) Europäisches Patentamt
European Patent Office
Office européen des brevets

(11) Veröffentlichungsnummer: **0 006 171**
**B1**

(12) # EUROPÄISCHE PATENTSCHRIFT

(45) Veröffentlichungstag der Patentschrift: **28.07.82**

(21) Anmeldenummer: **79101731.2**

(22) Anmeldetag: **01.06.79**

(51) Int. Cl.³: **C 07 D 271/06,** C 07 D 271/10, C 07 D 285/12, C 07 D 413/10, C 07 D 417/10, D 06 L 3/12, C 11 D 3/42, C 08 J 3/20

(54) **Neue Stilbenverbindungen, Verfahren zu deren Herstellung und deren Verwendung als optische Aufheller.**

(30) Priorität: **09.06.78 CH 6346/78**

(43) Veröffentlichungstag der Anmeldung: **09.01.80 Patentblatt 80/1**

(45) Bekanntmachung des Hinweises auf die Patenterteilung: **28.07.82 Patentblatt 82/30**

(84) Benannte Vertragsstaaten: **CH DE FR GB IT**

(56) Entgegenhaltungen:
**CH-A-506 583**
**CH-A-542 212**
**FR-A-1 479 071**
**FR-A-2 069 777**

(73) Patentinhaber: **HOECHST AKTIENGESELLSCHAFT, Postfach 80 03 20, D-6230 Frankfurt/Main 80 (DE)**

(72) Erfinder: **Günther, Dieter, Dr., Nachtigallenweg 1A, D-6233 Kelkheim (Taunus) (DE)**
Erfinder: **Eckes, Helmut, Dr., Feldbergblick 5, D-6239 Eppstein Taunus (DE)**
Erfinder: **Erckel, Rüdiger, Dr., Staufenstrasse 16, Eppstein/Taunus (DE)**
Erfinder: **Rösch, Günter, Hohlweg 17, D-6232 Bad Soden am Taunus (DE)**

EP 0 006 171 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Stilbenverbindungen, Verfahren zu deren Herstellung und deren Verwendung als optische Aufheller

Optische Aufheller aus der Reihe der Bis-Oxdiazolylstilbene sind bereits aus der FR-A 2 069 777 bekannt. Die dort beschriebenen Verbindungen sind jedoch in allen Fällen symmetrisch, d.h. die beiden Oxdiazol-Gruppen sind gleich. Es wurde nun gefunden, dass man die Aufhellwirkung derartiger Verbindungen erhöhen kann, wenn man den Stilben-Grundkörper durch zwei unterschiedliche Oxdiazolyl- bzw. Thiadiazolyl-Gruppen substituiert.

Gegenstand der Erfindung sind somit Verbindungen der allgemeinen Formel (1)

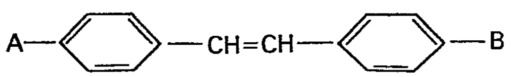 (1)

wobei A und B eine Gruppe der Formeln

a) 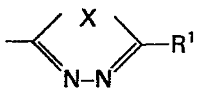 b) 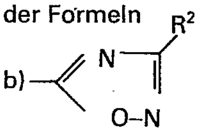

oder c) 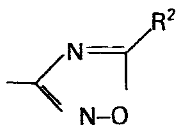

bedeuten mit der Massgabe, dass A und B nicht identisch sind und wobei $R^1$ einen Phenylring bedeutet, der durch ein oder zwei Chloratome, eine oder zwei $C_1$-$C_4$ Alkyl- oder $C_1$-$C_4$ Alkoxy-$C_1$-$C_4$-alkyl-gruppen, eine Phenyl-, Cyano-, Carbonsäure-, Carbo-$C_1$-$C_4$-alkoxy-, Carbonsäureamid-, Sulfonsäure-, Sulfonsäureamid- oder Sulfonsäure-$C_1$-$C_4$-alkylestergruppe substituiert sein kann,

$R^2$ die gleiche Bedeutung wie $R^1$ hat und zusätzlich jeweils eine $C_1$-$C_6$-Alkyl-, $C_1$-$C_6$-Chloralkyl-, $C_1$-$C_4$-Alkoxy-$C_1$-$C_4$-alkyl-, $C_1$-$C_4$-Hydroxyalkyl-, Benzylgruppe oder eine Gruppe der Formel $-(CH_2CH_2O)_n$-R' mit n=1 oder 2 und R'=Wasserstoff oder $C_1$-$C_4$-alkyl und x ein Sauerstoff- oder Schwefelatom bedeutet.

Insbesondere bevorzugt sind Verbindungen der Formel 1, worin A einen 1, 3, 4-Oxadiazol-2-; 1, 2, 4-Oxadiazol-5- oder 1, 2, 4-Oxadiazol-3-Ring, B einen 1, 2, 4-Oxadiazol-5- oder 1, 2, 4-Oxadiazol-3-Ring, $R^1$ Phenyl, mono- oder di-$C_1$-$C_2$-Alkylphenyl, $C_1$-$C_2$-Alkoxyphenyl, Chlorphenyl oder Dichlorphenyl bedeutet und $R^2$ die gleiche Bedeutung wie $R^1$ hat und zusätzlich jeweils $C_1$-$C_5$-Alkyl, $C_1$-$C_2$-Chloralkyl, $C_1$-$C_2$-Hydroxyalkyl, $C_1$-$C_2$-Alkoxyalkyl oder Benzyl bedeutet.

Gegenstand der Erfindung ist weiterhin ein Verfahren zur Herstellung der Verbindungen der Formel 1, das dadurch gekennzeichnet ist, dass man

a) wenn B eine Gruppe der Formel

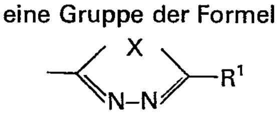 (2a)

bedeutet, entweder eine Säure der Formel

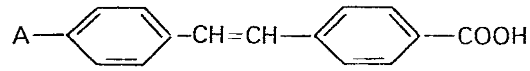 (3)

oder ein funktionelles Derivat dieser Säure mit einem Carbonsäurehydrazid der Formel

$H_2NNHCOR^1$ (4)

oder eine Säure der Formel

$R^1$–COOH (5)

oder ein funktionelles Derivat dieser Säure mit einer Verbindung der Formel

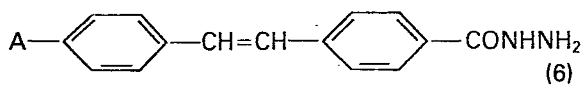 (6)

umsetzt und anschliessend mit einem wasserabspaltenden Mittel cyclisiert, wobei, wenn W ein Schwefelatom bedeutet, die Cyclisierung mit $P_2S_5$ erfolgt, oder

b) wenn B eine Gruppe der Formel

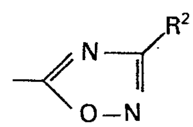 (2b)

bedeutet, eine Säure der Formel

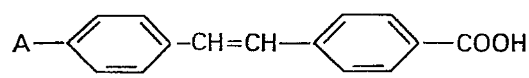 (3)

oder ein funktionelles Derivat dieser Säure mit einem Amidoxim der Formel

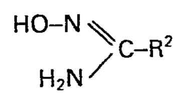 (7)

umsetzt und mit einem wasserabspaltenden Mittel cyclisiert oder

c) wenn B eine Gruppe der Formel

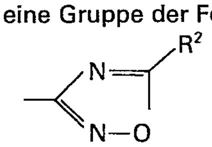 (2c)

bedeutet, eine Verbindung der Formel

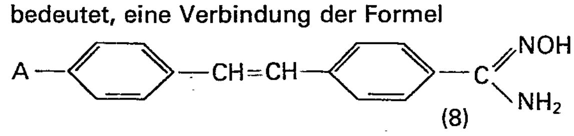 (8)

mit einem Carbonsäurechlorid der Formel

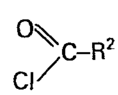

umsetzt und mit einem wasserabspaltenden Mittel cyclisiert, oder

d) eine Verbindung der Formel

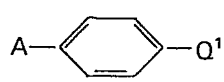 (10)

mit einer Verbindung der Formel

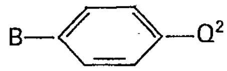

(11)

umsetzt, wobei eines der Symbole $Q^1$ oder $Q^2$ eine Aldehydgruppe und das andere Symbol eine Gruppe der Formeln

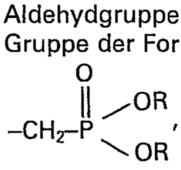,
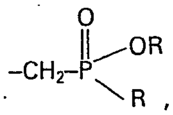,
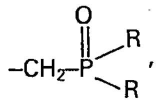,

oder 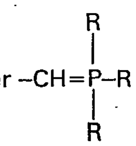

bedeutet, wobei R einen gegebenenfalls substituierten Alkylrest, mit vorzugsweise 1–6 C-Atomen, einen Arylrest, vorzugsweise Phenyl, einen Cycloalkylrest, vorzugsweise Cycloalkyl oder einen Aralkylrest, vorzugsweise Benzyl, darstellt.

Das gemeinsame Ausgangsprodukt für die Verfahren a) bis c) ist die Verbindung der Formel

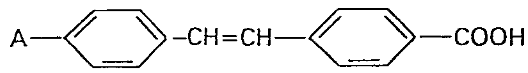

(3)

Je nach Bedeutung von A lässt sich diese Säure nach an sich bekannten Verfahren herstellen, wie in der folgenden Übersicht schematisch dargestellt.

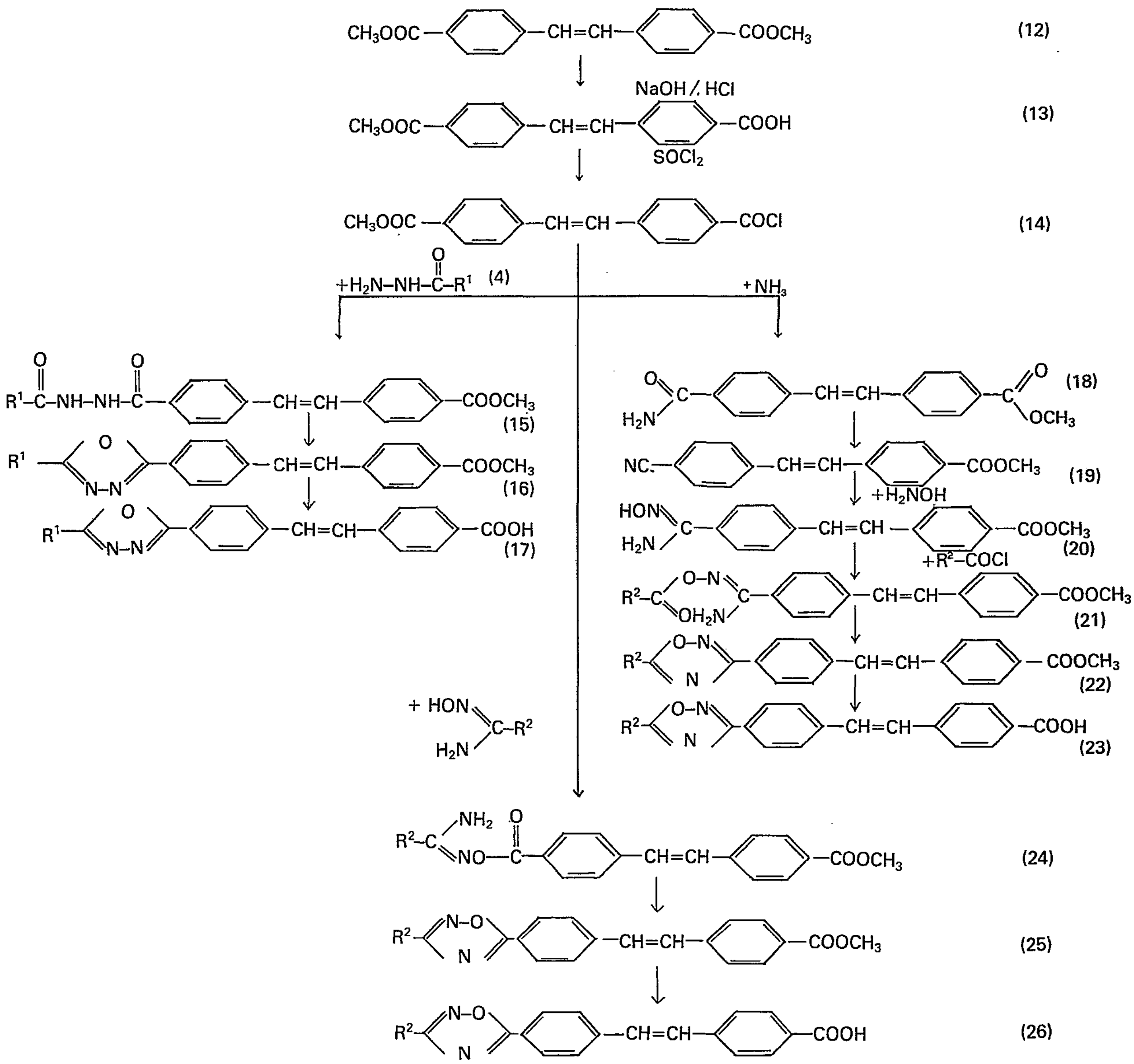


(12) (13) (14) (15) (16) (17) (18) (19) (20) (21) (22) (23) (24) (25) (26)

Die Umsetzung des Carbonsäurechlorids (14) zu den Acylderivaten (15), (18) und (24) erfolgt nach bekannten Verfahren, beispielsweise in einem organischen Lösungsmittel bei Raumtemperatur oder auch bei Temperaturen bis zu ca. 200° C, gegebenenfalls in Gegenwart von säurebindenden Mitteln. Der Ringschluss zu den Verbindungen (16) mit einem 1, 3, 4-Oxadiazolring erfolgt in Gegenwart von wasserabspaltenden Mitteln, wie zum Beispiel Thionylchlorid, Phosphoroxychlorid, Phosphorpentachlorid oder Phosphorpentoxid, gegebenenfalls in einem inerten

Lösungsmittel, bei Temperaturen von ca. 20–200° C. Der Ringschluss zu der Verbindung (25) und (22) mit einem 1, 2, 4-Oxadiazolring gelingt bereits durch Erhitzen des Vorprodukts in einem inerten Lösungmittel auf Temperaturen von 60–200° C. Die Verseifung der Carbomethoxygruppe zur freien Carbonsäuregruppe bei den Verbindungen (16), (25) und (22) erfolgt durch eine übliche Hydrolyse (DE-OS 12 94 917).

Die auf diese Weise erhältlichen Carbonsäuren (17), (26) bzw. (23) dienen direkt als Ausgangsverbindungen in den oben beschriebenen Verfahrensvarianten a), b) und c). Anstelle der freien Carbonsäuren kann man auch deren funktionelle Derivate, vorzugsweise die Säurechloride verwenden. Das als Ausgangsverbindung für die Verfahrensvariante c) dienende Amidoxim erhält man ebenfalls nach bekannten Verfahren aus diesen Nitrilen durch Addition von Hydroxylamin (DE-OS 27 09 924). Das Carbonsäurehydrazid für die Verfahrensvariante a) wird erhalten durch Umsetzung der Carbonsäuren oder Ester (16, 17, 25, 26) oder (22, 23) mit Hydrazin.

Die bei der Verfahrensvariante d) benötigten, Phosphoratome enthaltenden Ausgangsverbindungen werden erhalten, indem man nach bekannten Verfahren Halogenmethylverbindungen, vorzugsweise Chlormethyl- oder Brommethylverbindungen der Formeln

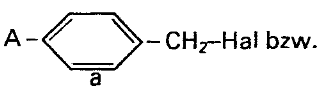 bzw. 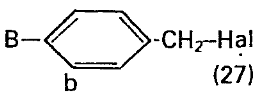 (27)

wobei A und B die oben genannten Bedeutungen haben und Hal vorzugsweise Chlor oder Brom bedeuten, mit Phosphorverbindungen der Formeln

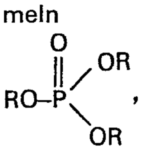 , 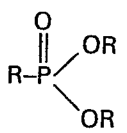 , 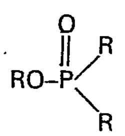 oder

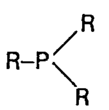

umsetzt, wobei R die oben angegebenen Bedeutungen hat. Die unmittelbar am Phorphor gebundenen Reste R bedeuten vorzugsweise Arylreste wir Phenyl, während die am Sauerstoff gebundenen Reste R vorzugsweise niedere Alkylgruppen bedeuten. Die Phosphoratome enthaltenden Ausgangsverbindungen für die Verfahrensvariante d) können auch erhalten werden, indem man die oben dargestellten Halogenmethylverbindungen mit P-Chlordiphenylphosphin und anschliessend mit einem Alkohol der Formel R–OH umsetzt, wobei R Wasserstoff oder einen Rest R wie oben definiert bedeuten. Die Umsetzung der ein Phosphoratom enthaltenden Ausgangsverbindungen mit den Verbindungen mit Aldehydfunktion gemäss Verfahrensvariante d) erfolgt vorteilhaft in indifferenten Lösungsmitteln. Als Beispiele hierfür seien Kohlenwasserstoffe wie Toluol und Xylol oder Alkohole wie Methanol, Äthanol, Isopropanol, Butanol, Glykole, Glykoläther wie 2-Methoxyäthanol, Hexanole, Cyclohexanol und Cyclooctanol, ferner Äther wie Diisopropyläther, Tetrahydrofuran und Dioxan sowie Dimethylsulfoxyd, Formamid und N-Methylpyrrolidon genannt. Besonders geeignet sind polare organische Lösungsmittel wie Dimethylformamid, N-Methylpyrrolidon und Dimethylsulfoxid. Auch in wässriger Lösung lassen sich einige der Umsetzungen durchführen.

Die Temperatur, bei welcher die Umsetzung durchgeführt wird, kann in weiten Grenzen schwanken. Sie wird bestimmt

α) durch die Beständigkeit des verwendeten Lösungsmittels gegenüber den Reaktionsteilnehmern, insbesondere gegenüber den stark basischen Alkaliverbindungen,

β) durch die Reaktivität der Kondensationspartner und

γ) durch die Wirksamkeit der Kombination Lösungsmittel-Base als Kondensationsmittel.

In der Praxis kommen hiernach im allgemeinen Temperaturen zwischen etwa 10 und 100° C in Betracht, insbesondere wenn Dimethylformamid oder Dimethylsulfoxid als Lösungsmittel verwendet werden. Der Temperatur-Vorzugsbereich liegt bei 20 bis 60° C. Es können jedoch unter Umständen auch höhere Temperaturen angewandt werden, wenn dies aus Gründen der Zeitersparnis erwünscht ist, oder ein weniger aktives, dafür aber billigeres Kondensationsmittel eingesetzt werden soll. Grundsätzlich sind somit auch Reaktionstemperaturen im Intervall von 10 bis 180° C möglich.

Als stark basische Alkaliverbindungen kommen vor allem die Hydroxyde, Amide und Alkoholate (vorzugsweise solche primärer 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z. B. Phenyl-lithium oder stark basische Amine (einschliesslich Ammoniumbasen, z. B. Trialkylammoniumhydroxyde) mit Erfolg verwendet werden.

Die vorstehend definierten neuen Verbindungen zeigen in gelöstem oder feinverteiltem Zustand eine mehr oder weniger ausgeprägte Fluoreszenz. Sie eignen sich deshalb zum optischen Aufhellen der verschiedensten synthetischen, halbsynthetischen oder natürlichen organischen Materialien oder Substanzen, welche solche organischen Materialien enthalten.

Hierfür seien beispielsweise, ohne dass durch die nachfolgende Übersicht irgendeine Beschränkung hierauf ausgedrückt werden soll, die nachfolgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung rekationsfähiger Gruppen erhaltene Produkte, beispielsweise Polymerisate auf Basis von α, β-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga) von Olefin-Kohlenwasserstoffen (wie z.B. Äthylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid), Polymerisationsprodukte die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiaminadipat), Maleinatharze, Melaminharze, deren Vorkondensate und Analoga, Polycarbonate, Silikone,

d) Polyadditionsprodukte wie Polyurethane (vernetzt und unvernetzt), Epoxidharze.

II. Halbsynthetische organische Materialien, z.B. Celluloseester verschiedener Veresterungsgrade (sogenanntes 2½-Acetat, Triacetat) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seiden, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilden vorliegen, d.h. beispielsweise als vorwiegend dreidimensional ausgedehnte Körper wie Platten, Profile, Spritzgussförmlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialen können beispielsweise als endlose Fäden (verstreckt oder unverstreckt), Stapelfasern, Flocken, Strangware, textile Fäden, Garne, Zwirne, Faservliese, Filze, Watten, Beflockungsgebilde oder als textile Gewebe oder textile Verbundstoffe, Gewirke sowie als Papiere, Pappen oder Papiermassen vorliegen.

Den erfindungsgemäss anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strängen, Geweben, Gewirken, Vliesen, beflockten Substraten oder Verbundstoffen vorliegen können, erfindungsgemäss optisch aufzuhellen sind, geschieht dies mit Vorteil in wässrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140° C, beispielsweise bei Siedtemperatur des Bades oder in deren Nähe (etwa 90° C), durchgeführt. Für die erfindungsgemässe Veredelung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden.

– Zugabe zu den Ausgangssubstanzen (z.B. Monomeren) oder Zwischenprodukten (z.B. Vorkondensation, Praepolymeren), d.h. vor oder während der Polymerisation, Polykondensation oder Polyaddition,

– Aufpudern auf Polymerisatschnitzel oder Granulate für Spinnmassen,

– Badfärbund von Polymerisatschnitzel oder Granulaten für Spinnmassen,

– Dosierte Zugabe zu Spinnschmelzen oder Spinnlösungen,

– Applikation auf Spinnkabel vor dem Verstrecken.

Die neuen optischen Aufhellmittel gemäss vorliegender Erfindung können beispielsweise auch in folgenden Anwendungsformen eingesetzt werden:

a) Mischungen mit Farbstoffen (Nuancierung) oder Pigmenten (Farb- oder insbesondere z.B. Weisspigmenten) oder als Zusatz zu Färbebändern, Druck-, Ätz- oder Reservepasten. Ferner auch zur Nachbehandlung von Färbungen, Drucken oder Ätzdrucken.

b) In Mischungen mit sogenannten «Carriern», Netzmitteln, Weichmachern, Quellmitteln, Antioxidantien, Lichtschutzmitteln, Hitzestabilisatoren, chemischen Bleichmitteln (Chlorit-Bleiche, Bleichbäder-Zusätze).

c) In Mischung mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie «wash-and-wear», «permanent-press», «noiron»), ferner Flammfest-, Weichgriff-Schmutzablöse («anti-soiling»)- oder Antistatisch-Ausrüstungen oder antimikrobiellen Ausrüstungen.

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu sogenannten «master batches»,

f) als Zusätze zu den verschiedensten industriellen Produkten, um dieselben marktfähiger zu machen (z.B. Aspektverbesserungen von Seifen, Waschmitteln, Pigmenten),

g) in Kombination mit anderen, optisch aufhellend wirkenden Substanzen,

h) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Scintillatoren, für verschiedene Zwecke photographischer Art, wie z.B. für elektrophotographische Reproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optische aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemässen Aufhellern zweckmässig in der Weise, dass man diese Fasern mit den wässrigen Dispersionen (gegebenenfalls auch Lösungen) der Aufhellmittel bei Temperaturen unter 75° C, z.B. bei Raumtemperatur, imprägniert und einer trockenen Wärmebehandlung bei Temperaturen über 100° C unterwirft, wobei es sich im allgemeinen empfiehlt, das Fasermaterial vorher noch bei mässig erhöhter Temperatur, z.B. bei mindestens 60° C bis etwa 130° C zu trocknen. Die Wärmebehandlung in trockenem Zustande erfolgt dann vorteilhaft bei Temperaturen zwischen 120 und 225° C, beispielsweise durch Erwärmen in einer Trockenkammer, durch Bügeln im angegebenen Temperaturintervall oder auch durch Behandeln mit trockenem überhitztem Wasserdampf. Die Trocknung und trockene Wärmebehandlung können auch unmittelbar nacheinander ausgeführt oder in einem einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,005 und 2, vorzugsweise 0,1 bis 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfatdecahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumpolyphosphate oder Alkalimetallsilikaten.

Die neuen optischen Aufhellmittel eignen sich auch besonders als Zusätze für Waschbäder oder zu Gewerbe- und Haushaltwaschmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässrige Dispersionen zugegeben. Zu Haushalt- oder gewerblichen Waschmitteln werden sie vorteilhaft in irgendeiner Phase des Herstellungsprozesses der Waschmittel, z.B. der sogenannten «Slurry» vor dem Zerstäuben dem Waschpulver oder bei der Vorbereitung flüssiger Waschmittelkombinationen zugesetzt. Die Zugabe kann sowohl in Form einer Lösung oder Dispersion in Wasser oder anderen Lösungsmitteln als auch ohne Hilfsmittel als trockenes Aufhellpulver erfolgen. Man kann die Aufhellmittel beispielsweise mit den waschaktiven Substanzen vermischen, verkneten oder vermahlen und so dem

fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulvern, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl-, oder -aminoacrylglycerinsulfonate, Phosphorsäureester von Fettalkoholen usw. in Frage. Als Aufbaustoffe, sogenannte «Builders», kommen z.B. Alkalipoly- und -polymethaphosphate, Alkalipyrophosphate, Alkalisalze der Carboxymethylcellulose und andere «soilredepositionsinhibitoren», ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein:

antistatische Mittel, rückfettende Hautschutzmittel wie Lanolin, Enzyme, Antimikrobika, Parfüme und Farbstoffe.

Die neuen optischen Aufheller haben den besonderen Vorteil, dass sie auch bei Gegenwart von Aktivchlorspendern, wie z.B. Hypochlorid, wirksam sind und ohne wesentliche Einbusse der Effekte in Waschbädern mit nichtionogenen Waschmitteln, wie z.B. Alkylphenolpolyglykoläthern, verwendet werden können.

Die erfindungsgemässen Verbindungen werden in Mengen von 0,005–1% oder mehr, bezogen auf das Gewicht des flüssigen des pulverförmigen, fertigen Waschmittels, zugesetzt. Waschflotten, die die angegebenen Mengen der beanspruchten optischen Aufheller enthalten, verleihen beim Waschen von Textilien aus Cellulosefasern, Polyamidfasern, hochveredelten Cellulosefasern, Polyesterfasern, Wolle etc. einen brillanten Aspekt am Tageslicht.

Die Waschbehandlung wird beispielsweise wie folgt durchgeführt:

Die angegebenen Textilien werden während 1 bis 30 Minuten bei 20 bis 100° C in einem Waschbad behandelt, das 1 bis 10g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1:3 bis 1:50 betragen. Nach dem Waschen wird wie üblich gespült und getrocknet. Das Waschbad kann als Bleichzusatz 0,2g/l Aktivchlor (z.B. als Hypochlorid) oder 0,1 bis 2g/l Natriumperborat enthalten.

In den folgenden Beispielen sind Prozente immer Gewichtsprozente, Schmelz- und Siedepunkte sind, sofern nicht anders vermerkt, unkorrigiert.

Beispiel 1

3,9g des Säurechlorids der Formel (28)

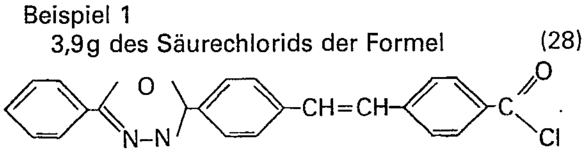

werden bei Raumtemperatur unter gutem Rühren in eine Lösung von 1,0g Acetamidoxim und 1,0ml Pyridin in 50ml Toluol eingetragen und nach 1 Std. bei Raumtemperatur, anschliessend 2 Stunden bei etwa 80° C gerührt. Nach Abkühlen und Absaugen des Produkts wäscht man halogenfrei und trocknet. Man erhält 3,7g der Acylaminoverbindung, welche zum Ringschluss in 40ml Dimethylformamid 4 Stunden am Rückfluss gekocht werden. Nach dem Klären mit Aktivkohle wird heiss filtriert. Aus der erhaltenen Lösung erhält man nach Absaugen und Waschen mit Methanol und Wasser 2,5g der Verbindung der Formel

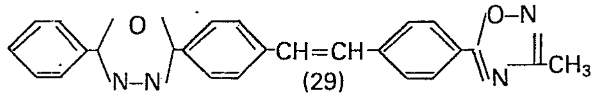

(29)

Aus o-Dichlorbenzol erhält man in Gegenwart von Bleicherde beim Umkristallisieren schwachgelbliche Kristalle vom Schmp. 223–224° C. Ultraviolett-Absorptionsmaximum bei $\lambda$ = 358nm, (gemessen in Dimethylformamid).

Das als Ausgangsmaterial verwendete Säurechlorid kann nach folgender Vorschrift hergestellt werden:

6,3g (20 mMol) der Verbindung der folgenden Formel

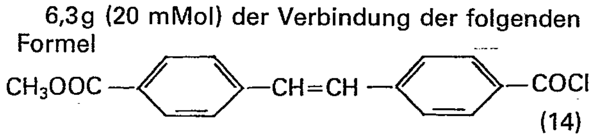

(14)

werden bei 0° C in 50ml Pyridin mit 2,8g (20 mMol) Benzoesäurehydrazid versetzt und anschliessend noch $1^h$ bei 0° C, $1^h$ bei Raumtemperatur und schliesslch $1^h$ bei Rückflusstemperatur gerührt. Die Reaktionsmischung wird nach Abkühlen auf 1l Eis-Wasser gegossen, abgesaugt und frei von Chloridionen gewaschen. Nach dem Trocknen erhält man 7,0g der Acylaminoverbindung.

Diese werden im 50ml Thionylchlorid $24^h$ auf Rückflusstemperatur erwärmt. Danach ist in einer Probe keine Acylaminoverbindung dünnschichtchromatographisch mehr nachweisbar. Der Thionylchloridüberschuss wird abdestilliert, zuletzt unter Anlegen eines leichten Vakuums. Der Rückstand wird $1^h$ mit 25ml Äthanol aufgekocht, abschliessend kalt abgesaugt und mit Äthanol und Wasser frei von Chloridionen gewaschen. Man erhält 5,8g der Verbindung der Formel

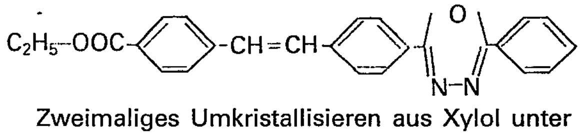

Zweimaliges Umkristallisieren aus Xylol unter Zuhilfenahme von Aktivkohle ergibt schwach gelbliche Kristalle vom Schmp. 241° C. Ultraviolett-Absorptionsmaximum bei $\lambda$ = 351nm (gemessen in Dimethylformamid).

4,0g dieses Esters werden in 30ml Dioxan mit 2ml 10N Natronlauge 2 Stdn. bei 85–90° C gerührt, anschliessend wird die Suspension mit 10ml Methanol verdünnt. Nach Abkühlen auf Raumtemperatur wird abgesaugt und mit Methanol gewaschen. Das feuchte Nutschgut wird in 100ml Dimethylformamid eingetragen und bei

100° C mit 5 ml konz. Salzsäure versetzt; nach Abkühlen wird abgesaugt, neutral gewaschen und getrocknet. Man erhält 3,0 g der Verbindung der Formel

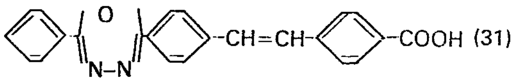
(31)

Aus dieser Carbonsäure erhält man durch 3stündiges Kochen in überschüssigem Thionylchlorid das Säurechlorid vom Fp. 224–240° C. Der Thionylchlorid-Überschuss wurde durch Abdestillieren, zuletzt unter Anlegen von Vakuum entfernt.

Beispiel 2

3,9 g des Säurechlorids der Formel

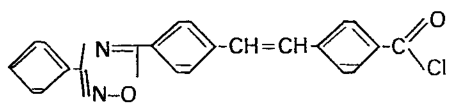
(32)

werden bei Raumtemperatur unter gutem Rühren in eine Lösung von 1,0 g Acetamidoxim und 1,0 ml Pyridin in 50 ml Toluol eingetragen und nach 1stündigem Rühren bei Raumtemperatur noch $2^h$ auf 75° C erwärmt. Das Produkt wird kalt abgesaugt, mit Methanol und Wasser gewaschen und getrocknet. Man erhält 3,4 g Acylaminoverbindung, die zum Ringschluss in 40 ml Dimethylformamid 4 Stdn. am Rückfluss gekocht werden. Die Reaktionsmischung wird mit Aktivkohle geklärt und heiss filtriert. Aus dem Filtrat erhält man nach Absaugen, Waschen und Trocknen 2,4 g der Verbindung der Formel

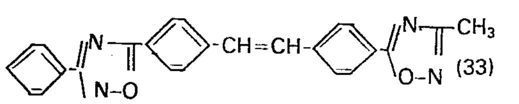
(33)

Aus o-Dichlorbenzol unter Zuhilfenahme von Bleicherde erhält man beim Umkristallisieren fast farblose Kristalle vom Schmp. 218–223° C Ultraviolett-Absorptionsmaximum bei λ = 358 nm (gemessen in Dimethylformamid).

Das als Ausgangsmaterial verwendete Säurechlorid kann nach folgender Vorschrift hergestellt werden:

In eine Lösung von 8,2 g (60 mMol) Benzamidoxim in 150 ml Pyridin werden bei Raumtemperatur 15,7 g (50 mMol) der Verbindung der Formel

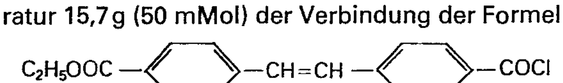
(14)

eingetragen, noch $1^h$ bei Raumtemperatur nachgerührt und anschliessend $1^h$ am Rückfluss gekocht.

Nach dem Abkühlen wird mit 1 l Eis-Wasser verdünnt, abgesaugt und frei von Chlorid-ionen gewaschen. Nach dem Trocknen erhält man 13,6 g Acylaminoverbindung.

Diese werden in 100 ml DMF $4^h$ am Rückfluss gekocht. In einer Probe ist dann keine Acylaminoverbindung mehr dünnschichtchromatographisch nachweisbar. Aus der Reaktionsmischung fallen beim Abkühlen 7,5 g der Verbindung der Formel

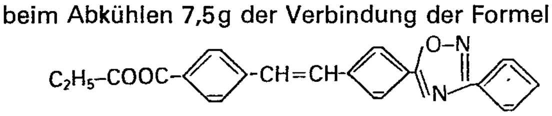

aus, die durch Umkristallisieren aus Xylol gereinigt werden. Man erhält fast farblose Kristalle vom Schmp. 235° C, Ultraviolett-Absorptionsmaximum bei λ = 345 nm (gemessen in Dimethylformamid).

4,0 g dieses Äthylesters werden in 30 ml Dioxan mit 2 ml 10N Natronlauge 10 Stdn. bei 75° C gerührt, anschliessend mit 10 ml Methanol verdünnt und nach Abkühlen auf Raumtemperatur abgesaugt. Das mit Methanol gewaschene, noch feuchte Nutschgut wird in 100 ml Dimethylformamid eingetragen und bei 100° C mit 5 ml konz. Salzsäure versetzt. Es wird kalt abgesaugt, neutral gewaschen und getrocknet. Die Ausbeute an Carbonsäure der Formel

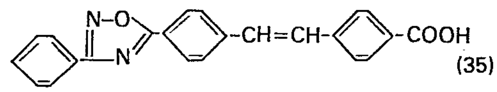
(35)

beträgt 2,9 g.

Diese Carbonsäure wird durch 5stündiges Kochen im überschüssigen Thionylchlorid in das Säurechlorid der Formel

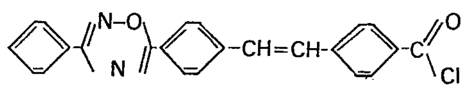
(36)

überführt. Nach Abdestillieren des Thionylchlorid-Überschusses, zuletzt mit Anlegen von Vakuum, besitzt das Säurechlorid den Siedepunkt von 212° C.

Beispiel 3:

Analog erhält man durch Umsetzen dieses Säurechlorids mit Benzoylhydrazid die Verbindung der Formel

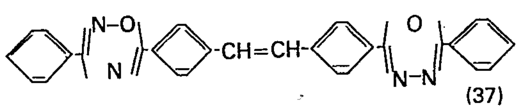
(37)

Schwach gelbliche Kristalle aus DMF vom Schmp. 277–278° C Ultraviolett-Absorptionsmaximum bei λ = 360 nm (gemessen in Dimethylformamid).

Beispiel 4:

Analog erhält man durch Umsetzen des obigen Säurechlorids mit 4-Phenylbenzoesäurehydrazid die Verbindung der Formel

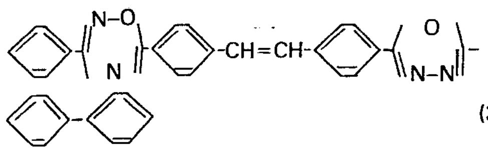
(38)

Leicht gelbliche Kristalle aus DMF vom Schmp. 293–294° C.

Beispiel 5:

Unter einer Stickstoffatmosphäre werden 2,3 g fein gepulvertes Kaliumhydroxid in 40 ml Dimethylformamid suspendiert. Unter leichter Kühlung wird eine Lösung von 2,6 g der Verbindung der Formel

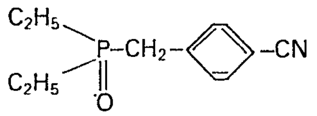

(39)

und 2,5g der Verbindung der Formel

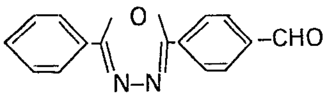

(40)

in 40ml Dimethylformamid eingetropft. Die Temperatur des Reaktionsgemisches soll dabei 25° C nicht übersteigen. Nach 20stündigem Rühren bei Raumtemperatur wird auf 100ml Eis-Wasser, welches 4ml konz. Salzsäure enthält, gegossen, kurz auf 90° C erwärmt und nach dem Erkalten abgesaugt. Nach Neutralwaschen und Trocknen erhält man 2,6g der Verbindung der Formel

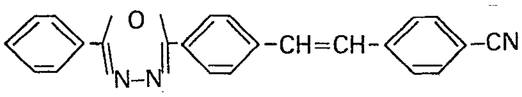

(41)

die in Schwefelsäure zur Verbindung (31) verseift wird. Die Verbindung der Formel

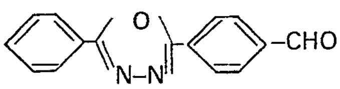

(42)

erhält man wie folgt:

23,6g 2-Phenyl-5-(4-methylphenyl)-1, 3, 4-oxadiazol werden in 200ml absolutem Tetrachlorkohlenstoff unter leichtem Erwärmen gelöst. Es werden 17,8g N-Bromsuccinimid und 0,2g Azoisobuttersäurenitril zugegeben und 4^h am Rückfluss gekocht. Es wird heiss abfiltriert, aus dem Filtrat kristallisieren beim Abkühlen 20,2g der Verbindung der Formel

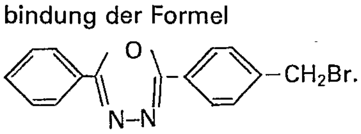

(43)

Aus Essigsäureäthylester erhält man farblose Kristalle vom Schmp. 170–172° C.

15,7g dieser Bommethylverbindung werden in 250ml Chloroform gelöst und mit einer Lösung von 21g Urotropin in 250ml Chloroform vereinigt. Die Mischung wird 24^h bei Rückflusstemperatur gehalten, der ausgefallene Niederschlag wird kalt abgesaugt, mit Chloroform gewaschen und getrocknet. Der Rückstand wird in fein pulverisierter Form in einem Gemisch aus 135ml Eisessig und 135ml Wasser 2^h bei Rückflusstemperatur gehalten. Beim Abkühlen kristallisieren 7,2g (57% d.Th.) der Verbindung der Formel

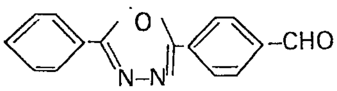

(44)

aus. Aus Äthanol erhält man farblose Kristalle vom Schmp. 275–280° C.

Beispiel 6:

2,2g (20 mMol) Chloracetamidoxim werden bei 10°–18° C in 30l N-Methylpyrrolidon vorgelegt, 3,9g (10 mMol) des Säurechlorids (38) eingetragen, 1 Std. bei 10°–15° C und 1h bei 35°–40° C nachgerührt, eiskalt abgesaugt, mit Methanol gewaschen und trocken gesaugt. Der Rückstand wird in 20l Dimethylformamid eingetragen, anschliessend 2 Std. am Rückfluss gerührt und auf 25° C abgekühlt. Es wird über 3^h Dimethylamin bei dieser Temperatur eingeleitet und 3 Std. bei 50° C nachgerührt. Es wird kalt abgesaugt, mit Methanol und Wasser gewaschen und getrocknet. Man erhält 2,4g (53,6% d.Th.) der Verbindung

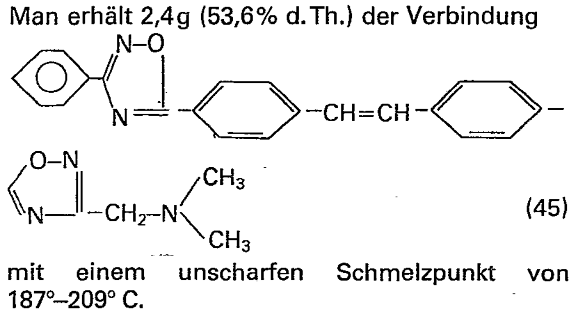

(45)

mit einem unscharfen Schmelzpunkt von 187°–209° C.

Beispiel 7:

2g (4,5 mMol) der Verbindung (45) werden in 25ml Dioxan heiss gelöst, 0,45ml (5 mMol) Dimethylsulfat zugetropft und 2 Std. am Rückfluss nachgerührt. Nach dem Abkühlen wird abgesaugt mit Dioxan und Aceton gewaschen und getrocknet. Man erhält 1,9g (73,6% d.Th.) der Verbindung

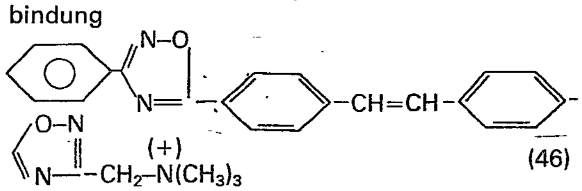

(46)

welche nach Umkristallisieren aus Dimethylformamid bei 268–270° C schmilzt.

Beispiel 8:

Arbeitet man wie in Beispiel 6 und setzt an Stelle des Säurechlorids (36) das Säurechlorid (28) ein, so erhält man in 50% Ausbeute die Verbindung der Formel

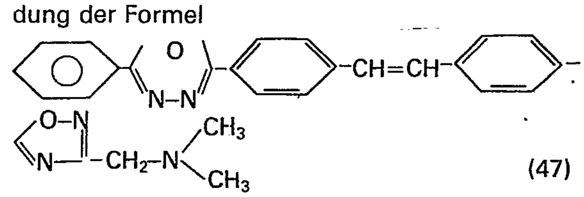

(47)

welche bei 188–190° C schmilzt.

Beispiel 9:

Arbeitet man wie in Beispiel 7 nur mit der Verbindung (47) an Stelle der Verbindung (45) so erhält man in 77% Ausbeute die Verbindung

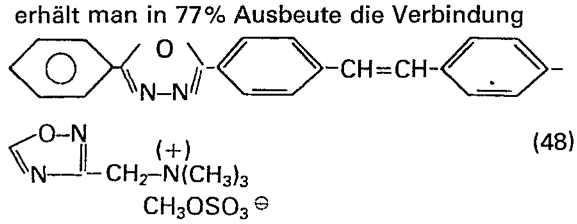

(48)

welche bei 287° C schmilzt.

Beispiel 10:

Analog Beispiel 1 erhält man die Verbindung

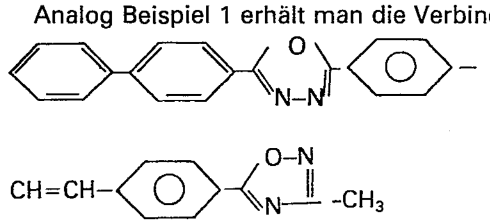

(49)

vom Schmelzpunkt 270° C.

**Patentansprüche**

1. Verbindungen der allgemeinen Formel (1)

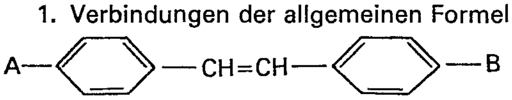

(1)

wobei A und B eine Gruppe der Formeln

a) 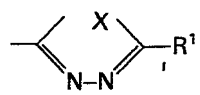 b) 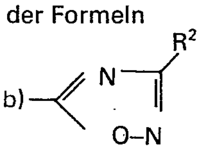

oder c) 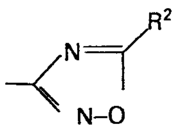

bedeuten mit der Massgabe, dass A und B nicht identisch sind und wobei $R^1$ einen Phenylring bedeutet, der durch ein oder zwei Chloratome, eine oder zwei $C_1$-$C_4$ Alkyl- oder $C_1$-$C_4$ Alkoxy-$C_1$-$C_4$-alkyl-gruppen, eine Phenyl-, Cyano-, Carbonsäure-, Carbo-$C_1$-$C_4$-alkoxy-, Carbonsäureamid-, Sulfonsäure-, Sulfonsäureamid- oder Sulfonsäure-$C_1$-$C_4$-alkylestergruppe substituiert sein kann,

$R^2$ die gleiche Bedeutung wie $R^1$ hat und zusätzlich jeweils eine $C_1$-$C_6$-Alkyl-, $C_1$-$C_6$-Chloralkyl-, $C_1$-$C_4$-Alkoxy-$C_1$-$C_4$-alkyl-, $C_1$-$C_4$-Hydroxyalkyl-, Benzylgruppe oder eine Gruppe der Formel $-(CH_2CH_2O)_n$-R' mit n=1 oder 2 und R'=Wasserstoff oder $C_1$-$C_4$-alkyl und x ein Sauerstoff- oder Schwefelatom bedeutet.

2. Verbindungen der Formel 1 nach Anspruch 1, worin A einen 1, 3, 4-Oxadiazol-2-, 1, 2, 4-Oxadiazol-5- oder 1, 2, 4-Oxadiazol-3-Ring, $R^1$ Phenyl, mono- oder di-$C_1$-$C_2$-Alkylphenyl, $C_1$-$C_2$-Alkoxyphenyl, Chlorphenyl oder Dichlorphenyl bedeutet und $R^2$ die gleiche Bedeutung wie $R^1$ hat und zusätzlich jeweils $C_1$-$C_5$-Alkyl, $C_1$-$C_2$-Chloralkyl, $C_1$-$C_2$-Hydroxyalkyl, $C_1$-$C_2$-Alkoxyalkyl oder Benzyl bedeutet.

3. Verfahren zur Herstellung der Verbindungen nach Anspruch 1 und 2, dadurch gekennzeichnet, dass man

a) wenn B eine Gruppe der Formel

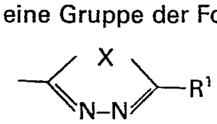

(2a)

bedeutet, entweder eine Säure der Formel

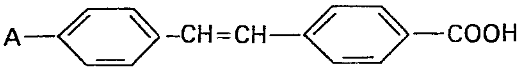

(3)

oder ein funktionelles Derivat dieser Säure mit einem Carbonsäurehydrazid der Formel

$$H_2NNHCOR^1 \quad (4)$$

oder eine Säure der Formel

$$R^1\text{–}COOH \quad (5)$$

oder ein funktionelles Derivat dieser Säure mit einer Verbindung der Formel

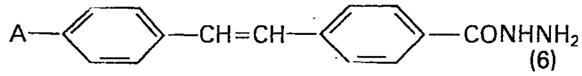

(6)

umsetzt und anschliessend mit einem wasserabspaltenden Mittel cyclisiert, wobei, wenn X ein Schwefelatom bedeutet, die Cyclisierung mit $P_2S_5$ erfolgt, oder

b) wenn B eine Gruppe der Formel

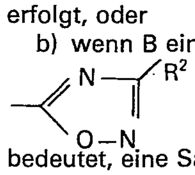

(2b)

bedeutet, eine Säure der Formel

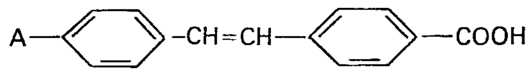

(3)

oder ein funktionelles Derivat dieser Säure mit einem Amidoxim der Formel

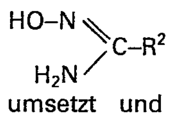

(7)

umsetzt und mit einem wasserabspaltenden Mittel cyclisiert oder

c) wenn B eine Gruppe der Formel

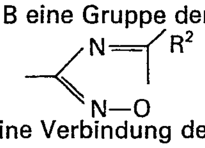

(2b)

bedeutet, eine Verbindung der Formel

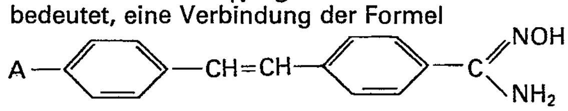

mit einem Carbonsäurechlorid der Formel (8)

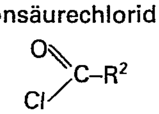

umsetzt und mit einem wasserabspaltenden Mittel cyclisiert, oder

d) eine Verbindung der Formel

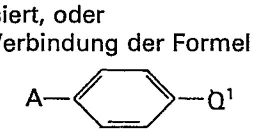

(10)

mit einer Verbindung der Formel

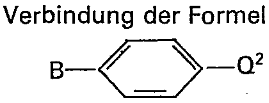

(11)

umsetzt, wobei eines der Symbole $Q^1$ oder $Q^2$ eine Aldehydgruppe und das andere Symbol eine Gruppe der Formeln

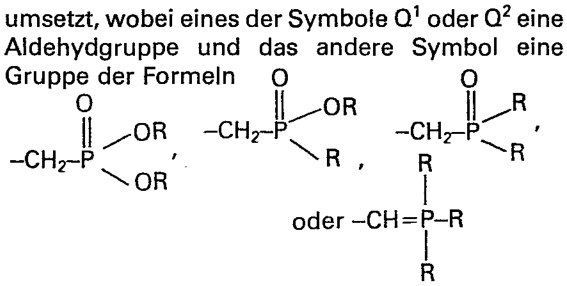

oder

bedeutet, wobei R einen gegebenenfalls substituierten Alkylrest, mit vorzugsweise 1–6 C-Atomen, einen Arylrest, vorzugsweise Phenyl, einen Cycloalkylrest, vorzugsweise Cyclohexyl oder einen Aralkylrest, vorzugsweise Benzyl, darstellt.

4. Verwendung der Verbindungen gemäss Anspruch 1 und 2 als optische Aufheller.

## Claims

1. Compounds of the general formula (1)

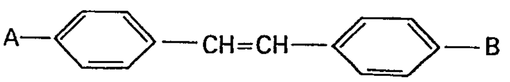 (1)

in which A and B represent a group of the formulae

a) 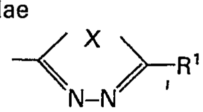 b) 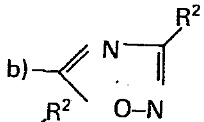

or c) 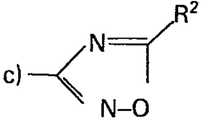

with the proviso that A and B do not represent the same heterocycle, and in which $R^1$ is a phenyl ring which may be substituted by 1 or 2 chlorine atoms, 1 or 2 $C_{1-4}$-alkyl group(s) or $C_{1-4}$-alkoxy-$C_{1-4}$-alkyl group(s), phenyl, cyano, carboxylic acid, carbo-$C_{1-4}$-alkoxy, carboxylic acid amide, sulfonic acid, sulfonic acid amide or sulfonic acid-$C_{1-4}$-alkyl ester, and $R^2$ is defined as $R^1$ and additionally represents $C_{1-6}$-alkyl, $C_{1-6}$-chloroalkyl, $C_{1-4}$-alkoxy-$C_{1-4}$-alkyl, $C_{1-4}$-hydroxyalkyl and benzyl, respectively, or a group of the formula $-(CH_2CH_2O)_n-R'$ with n being 1 or 2 and R' being hydrogen or $C_{1-4}$-alkyl and X is an oxygen or sulphur atom.

2. Compounds of the formula 1 as claimed in claim 1, in which A is an 1, 3, 4-oxadiazole-2 ring, an 1, 2, 4-oxadiazole-5 or an 1, 2, 4-oxadiazole-3 ring, $R^1$ is phenyl, mono- or di-$C_{1-2}$-alkylphenyl, $C_{1-2}$-alkoxyphenyl, chlorophenyl or dichlorophenyl and $R^2$ is defined as $R^1$ and additionally represents $C_{1-5}$-alkyl, $C_{1-2}$-chloroalkyl, $C_{1-2}$-hydroxyalkyl, $C_{1-2}$-alkoxyalkyl and benzyl, respectively.

3. Process for preparing the compounds as claimed in claims 1 and 3, which comprises

a) if B represents a group of the formula

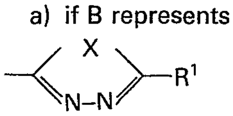 (2a)

reacting either an acid of the formula

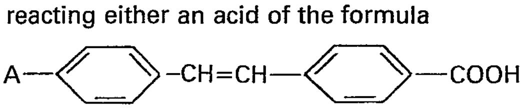 (3)

or a functional derivate of this acid with a carboxylic acid hydrazide of the formula

$H_2NNHCOR^1$ (4)

or an acid of the formula

$R^1-COOH$ (5)

or a functional derivative of this acid with a compound of the formula (6)

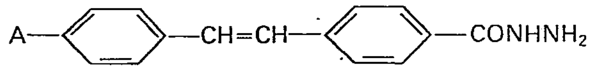

and thereafter cyclisizing the product obtained with an agent splitting off water, in which process – if X is a sulfur atom – the cyclization is effected with $P_2S_5$, or

b) if B represents a group of the formula

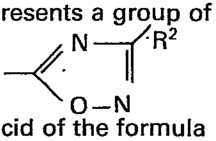 (2b)

reacting an acid of the formula

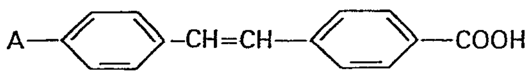 (3)

or a functional derivative of this acid with an amidoxime of the formula

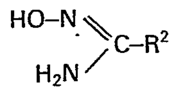 (7)

and cyclisizing the product obtained with an agent splitting off water, or

c) if B stands for a group of the formula

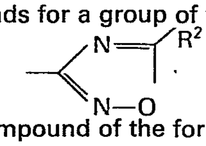

reacting a compound of the formula

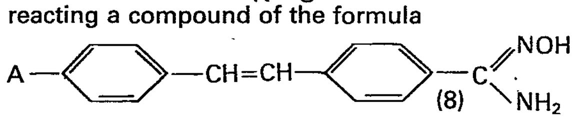 (8)

with a carboxylic acid chloride of the formula

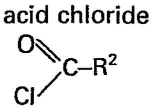

and cyclisizing the product obtained with an agent splitting off water, or

d) reacting a compound of the formula

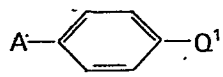 (10)

with a compound of the formula

B Q[2] 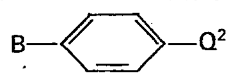 (11)

in which one of the symbols $Q^1$ and $Q^2$ represents an aldehyde group and the other symbol represents a group of the formulae

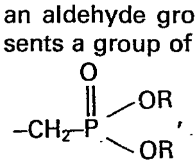, 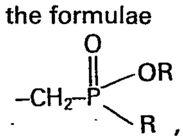, 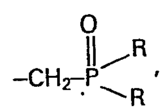,

or 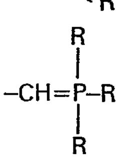

in which R stands for an optionally substituted alkyl radical, with preferably from 1 to 6 carbon atoms, aryl, preferably phenyl, cycloalkyl, preferably cyclohexyl, or aralkyl, preferably benzyl.

4. Use of the compounds of claims 1 and 2 as optional brighteners.

**Revendications**

1. Composés répondant à la formule générale (1)

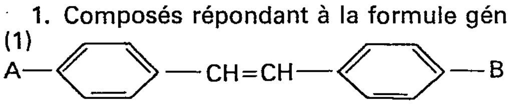 (1)

dans laquelle A et B, qui, dans un même composé, sont obligatoirement différents l'un de l'autre, représentent chacun un radical répondant à l'une des formules suivantes:

a) b) et c) 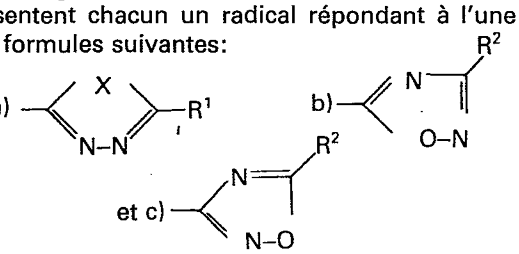

formules dans lesquelles

$R^1$ représente un noyau phénylique éventuellement porteur d'un ou deux atomes de chlore, d'un ou deux radicaux alkyles en $C_1$-$C_4$ ou ($C_1$-$C_4$) alcoxy-($C_1$–$C_4$)alkyles, ou d'un radical phényle, cyano, carboxy, alcoxycarbonyle en $C_2$-$C_5$, carbamoyle, sulfo, sulfamoyle ou alcoxysulfonyle en $C_1$–$C_4$, a la même signification que $R^1$ et peut en outre représenter un alkyle en $C_1$-$C_6$, un chloralkyle en $C_1$-$C_6$, un ($C_1$-$C_4$)alcoxy-($C_1$-$C_4$)alkyle, un hydroxy-alkyle en $C_1$-$C_4$ ou un benzyle ou un radical $-(CH_2CH_2O)_n$-R' dans lequel n est égal à 1 ou à 2 et R' désigne l'hydrogène ou un alkyle en $C_1$-$C_4$, et

X représente un atome d'oxygène ou de soufre.

2. Composés de formule 1 selon la revendication 1, dans lesquels A représente un radical oxadiazole-1, 3, 4 yle-2, oxadiazole-1, 2, 4 yle-5 ou oxadiazole-1, 2, 4 yle-3, $R^1$ représente un radical phényle, monoalkylphényle à alkyle en $C_1$ ou $C_2$, dialkylphényle à alkyles en $C_1$ ou $C_2$, alcoxyphényle à alcoxy en $C_1$ ou $C_2$, chlorophényle ou dichlorophényle, et $R^2$ a la même signification que $R^1$ et peut en outre représenter un alkyle en $C_1$-$C_5$, un chloralkyle en $C_1$ ou $C_2$, un hydroxyalkyle en $C_1$ ou $C_2$, un alcoxyalkyle à alkyle en $C_1$ ou $C_2$ ou un benzyle.

3. Procédé de préparation de composés selon l'une des revendication 1 et 2, procédé caractérisé en ce que:

a) lorsque B représente un radical de formule 2a

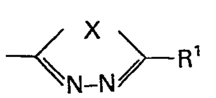 (2a)

on fait réagir soit un acide de formule 3

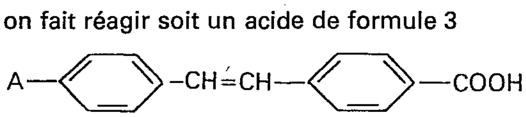 (3)

ou un dérivé fonctionnel de cet acide, avec un carbohydrazide de formule 4

$$H_2NNCOR^1 \quad (4)$$

soit un acide de formule 5

$$R^1\text{–}COOH \quad (5)$$

ou un dérivé fonctionnel de cet acide, avec un composé de formule 6

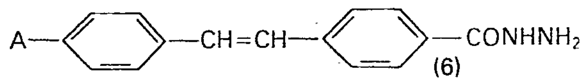

puis on cyclise au moyen d'un agent déshydratant, cette cyclisation étant effectuée à l'aide de $P_2O_5$ dans le cas ou X doit représenter un atome de soufre, ou

b) lorsque B est un radical de formule 2b

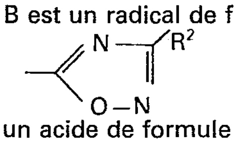 (2b)

ou fait réagir un acide de formule 3

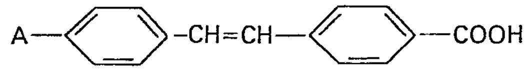 (3)

ou un dérivé fonctionnel de cet acide, avec une carboxamideoxime de formule 7

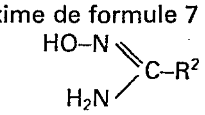 (7)

et on cyclise au moyen d'un agent déshydratant, ou

c) lorsque B représente un groupe de formule

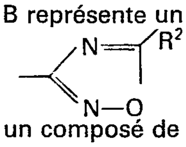

on fait réagir un composé de formule 8

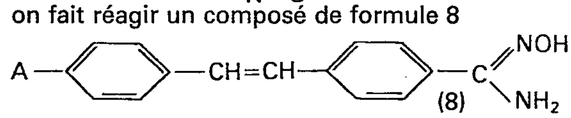

avec un chlorure d'acide carboxylique de formule

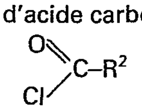

et on cyclise au moyen d'un agent déshydratant, ou

d) on fait réagir un composé de formule 10

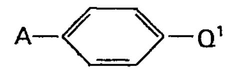 (10)

avec un composé de formule 11

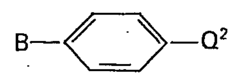 (11)

formules dans lesquelles l'un des symboles $Q^1$ et $Q^2$ représente un radical formyle (carbaldéhyde) et l'autre un radical répondant à l'une des formules suivantes:

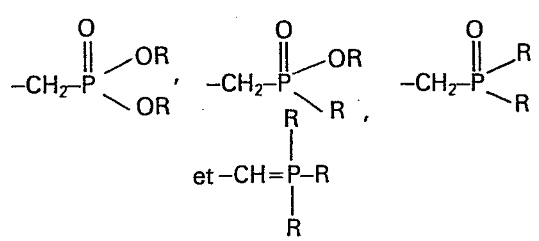

dans lesquelles R représente un radical alkyle éventuellement substitué, qui contient de préférence de 1 à 6 atomes de carbone, un radical aryle, de préférence phényle, un radical cycloalkyle, de préférence cyclohexyle, ou un radical aralkyle, de préférence benzyle.

4. Application des composés selon l'une des revendications 1 et 2 comme azureurs optiques.